# EUROPÄISCHE PATENTSCHRIFT

(11) **EP 0 603 893 B1**
(45) Veröffentlichungstag und Bekanntmachung des Hinweises auf die Patenterteilung: **08.08.2001**
(21) Anmeldenummer: 93120812.8
(22) Anmeldetag: 23.12.1993
(51) Int. Cl.: C07D 239/60

(54) **Verfahren zur Herstellung von 2-Ethoxy-4,6-dihydroxypyrimidin oder dessen Alkalisalz**
Process for the preparation of 2-ethoxy-4,6-dihydroxy pyrimidin or their alkaline salts
Procédé de préparation de 2-éthoxy-4,6-dihydroxypyrimidine ou leurs sels alcalins

(30) Priorität: 24.12.1992 DE 4244132; 15.09.1993 DE 4331223
(43) Veröffentlichungstag der Anmeldung: 29.06.1994
(73) Patentinhaber: SKW Trostberg Aktiengesellschaft, 83308 Trostberg (DE)
(72) Erfinder: Hintermaier, Helmut, Dr., D-83308 Trostberg (DE); Maier, Ursula, D-83342 Tacherting (DE); Weiss, Stefan, Dr., D-83308 Trostberg (DE)
(74) Vertreter: Huber, Bernhard, Dipl.-Chem.

(56) Entgegenhaltungen:
- DE-A- 1 948 370
- DE-A- 2 523 324

## Beschreibung

Gegenstand der vorliegenden Erfindung ist ein Verfahren zur Herstellung von 2-Ethoxy-4,6-dihydroxypyrimidin (bzw. dessen tautomerer Formen) oder dessen Alkalisalz.

Pyrimidin-Derivate finden als Zwischen- und Endprodukt vielfältigen Einsatz vor allem im Bereich der Pharmazie und Agrochemie. So ist deren Verwendung in Fungiziden oder Herbiziden hinlänglich bekannt. In diesem Zusammenhang sind auch substituierte Alkoxypyrimidine als Zwischenprodukte von Interesse.

Die Synthese von 4,6-Dihydroxy-2-methoxypyrimidin gelang S. Basterfield und E.C. Powell (Canad. J. Res. 1 265f (1929)) durch Umsetzung von Malonsäuredimethylester mit O-Methylisoharnstoff und H. Bretschneider, J. Dehler und W. Klötzer (Mh. Chem. 95, 207 (1964)) aus dem Hydrochlorid von O-Methylisoharnstoff durch Reaktion mit Malonsäuredimethylester in Gegenwart von Natriummethylat. C.J. Moye (Aust. J. Chem. 17, 1309 f (1964)) erhält das 4,6-Dihydroxy-2-methoxypyrimidin durch Umsetzung von O-Methylisoharnstoff-monomethylsulfatmitMalonsäurediethylester und Natriummethylat.

Die Übertragung der aus der Literatur bekannten Laborsynthesen von 4,6-Dihydroxy-2-methoxypyrimidin auf die Herstellung der analogen 2-Ethoxyverbindung im technischen Maßstab bereitet jedoch folgende Schwierigkeiten:

Entsprechend dem Verfahren von C.J. Moye müßte von Diethylsulfat und Harnstoff ausgegangen werden. Die Umsetzung von Diethylsulfat mit Harnstoff zum O-Ethylisoharnstoff-monoethylsulfat ist im technischen Maßstab jedoch nicht oder nur sehr schwierig durchführbar.
- Beim Erhitzen von Diethylsulfat mit Harnstoff kommt es zu einer spontan ablaufenden, stark exothermen Reaktion, die im technischen Maßstab nicht beherrscht werden kann.
- Diethylsulfat ist eine sehr giftige, krebserzeugende Substanz, die besondere arbeitshygienische und umweltschützende Maßnahmen erfordert.

In Analogie zu den Verfahren von Bretschneider und Mitarbeiter wird zur Herstellung von 2-Ethoxy-4,6-dihydroxypyrimidin O-Ethylisoharnstoff-hydrochlorid benötigt, dessen technische und wirtschaftliche Herstellung sehr schwierig ist.
- Aufgrund der guten Löslichkeit von O-Ethylisoharnstoff-hydrochlorid in Ethanol werden keine guten Ausbeuten erhalten vgl. Beispiel 3 der DE-OS 19 48 370.
- aufgrund der starken Korrosionsfähigkeit von O-Ethylisoharnstoff-hydrochlorid sind zu seiner Isolierung und Trocknung sehr teure Spezialapparate notwendig;
- festes O-Ethylisoharnstoff-chlorid spaltet leicht Ethylchlorid ab.

Während O-Methylisoharnstoff-Salze breite Anwendung zur Synthese von Heterocyclen finden, sind Synthesen von heterocyclischen 5- und 6-Ringen durch Umsetzung von C₃-Bausteinen mit O-Ethylisoharnstoff-Salzen in Gegenwart von einem Natriumalkoholat nicht bekannt. O-Ethylisoharnstoff ist bei Heterocyclen-Synthesen gegenüber C₃-Bausteinen weniger reaktionsfähig als O-Methylisoharnstoff, was auf sterischen Gründen beruht.

Nach dem Stand der Technik muß erwartet werden, daß die Synthese von 2-Ethoxy-4,6-dihydroxypyrimidin bzw. dessen Alkalisalz in Anlehnung an die bekannten Herstellungsverfahren durch Umsetzung von einem Malonsäureester mit einem O-Ethylisoharnstoff-Salz in Gegenwart von Natriumalkoholat im technischen Maß in wirtschaftlicher Weise nicht möglich ist. Diese Annahme wird durch die Tatsache bestätigt, daß sich in der deutschen Offenlegungsschrift DE 25 23 324 und in der belgischen Patentschrift BE 833 082, in denen das 2-Ethoxy-4,6-dihydroxypyrimidin erwähnt wird, keinerlei Angaben über das Herstellungsverfahren, die erzielte Ausbeute und die Reinheit des Produktes finden. Ferner wird die Verbindung weder durch eine Elementaranalyse noch durch Angaben zum Schmelzpunkt oder ein Spektrum charakterisiert.

Es hat sich daher die Aufgabe gestellt, ein Verfahren bereitzustellen, mit dessen Hilfe es möglich ist, 2-Ethoxy-4,6-dihydroxypyrimidin oder dessen Alkalisalz ohne die vorgenannten Probleme im technischen Maßstab unter wirtschaftlichen Bedingungen und einem möglichst geringen Anfall von Nebenprodukten und Reststoffen zu synthetisieren.

Es hat sich überraschenderweise gezeigt, dass mit Hilfe der vorliegenden Erfindung 2-Ethoxy-4,6-dihydroxypyrimidin oder ein Salz davon, technisch in wirtschaftlicher Weise mit sehr hoher Reinheit, guten Ausbeuten und einer günstigen Ökobilanz hergestellt werden kann.

Gegenstand der vorliegenden Erfindung ist ein Verfahren zur Herstellung von 2-Ethoxy-4,6-dihydroxypyrimidin bzw. dessen Salz gemäß Anspruch 1. Erfindungsgemäß wird hierzu im Verfahrensschritt a) O-Ethylisoharnstoff oder ein Salz davon aus Cyanamid oder Chlorformamidinium-Salzen-Salzen intermediär erzeugt und anschließend wird im zweiten Verfahrensschritt (b) der erzeugte O-Ethylisoharnstoff oder dessen Salz in situ mit einem Malonsäuredialkylester und einem Alkoholat oder mit einem Salz des Malonsäuredialkylesters zum Salz des 2-Ethoxy-4,6-dihydroxypyrimidins bei -10 bis 180 °C reagieren gelassen.

Der dritte Verfahrensschritt (c) besteht darin, daß gegebenenfalls aus dem Salz des 2-Ethoxy-4,6-dihydroxypyrimidins bzw. seinen tautomeren Formen das 2-Ethoxy-4,6-dihydroxypyrimidin durch Zugabe einer Säure in Freiheit gesetzt wird.

Die Herstellung des O-Ethylisoharnstoff-Salzes kann erfindungsgemäß durch Umsetzung von Cyanamid mit Ethanol in Gegenwart einer Mineralsäure oder von wasserfreiem Chlorwasserstoff oder einer starken organischen Säure erfolgen. Vorzugsweise wird diese Umsetzung im Temperaturbereich -10 bis +90°C durchgeführt. Als Mineralsäuren setzt man beispielsweise konzentrierte Schwefelsäure (> 95 %) ein. Eine weitere bevorzugte Verfahrensvariante benutzt als Mineralsäure eine Mischung aus technischer Schwefelsäure und rauchender Schwefelsäure. Aber auch rauchende Schwefelsäure selbst ist für die genannte Umsetzung geeignet. Die SO₃-Menge der Schwefelsäure sollte in jedem Fall so bemessen sein, daß die mit den anderen Reaktionspartnern, wie Ethanol, eingetragene Wassermenge vollständig zu Schwefelsäure umgesetzt wird.

Als starke organische Säuren kommen Säuren mit einem pKₐ von ≤ 3 in Frage. Vorzugsweise werden Sulfonsäuren, z.B. Methansulfonsäure, Benzolsulfonsäure und p-Toluolsulfonsäure oder Halogenessigsäuren, z.B. Trichloressigsäure, verwendet.

Pro 1 Mol Cyanamid werden 1 bis 2 Val Mineralsäure, vorzugsweise 1 bis 1,2 Val Mineralsäure, oder 1 bis 2 Mol Chlorwasserstoff, vorzugsweise 1 bis 1,2 Mol Chlorwasserstoff, eingesetzt. Ethanol wird im allgemeinen im Überschuß angewendet, wobei das überschüssige Ethanol als Lösungsmittel dient. Das Verhältnis Cyanamid zu Ethanol kann in weiten Grenzen variiert werden. Die Mindestmenge Ethanol muß jedoch so bemessen werden, daß das Reaktionsgemisch im technischen Maßstab noch in einem rühr- und pumpfähigen Zustand vorliegt. Im allgemeinen werden pro 1 Mol Cyanamid 2,5 bis 6,5 Mol Ethanol verwendet. Die Reaktionstemperatur hängt von der Stärke der Säure ab. Die Umsetzung erfolgt vorzugsweise im Temperaturbereich -10 bis +50°C, insbesondere bei 5 bis 30°C.

Erfindungsgemäß kann O-Ethylisoharnstoff-hydrochlorid überraschenderweise auch durch Umsetzung von Cyanamid mit Ethanol in Gegenwart einer Chlorwasserstoff-abspaltenden Verbindung wie z.B. Siliciumtetrachlorid oder Thionylchlorid hergestellt werden. Die Verwendung einer HCl-abspaltenden Verbindung hat den Vorteil, daß nicht mit korrosivem Chlorwasserstoff gearbeitet werden muß. Die Umsetzung erfolgt vorzugsweise bei Temperaturen im Bereich von -10 bis +90°C, wobei Reaktionstemperaturen von -5 bis +40°C besonders vorteilhaft sind. Die HCl-abspaltende Verbindung wird üblicherweise in einer solchen Menge eingesetzt, daß pro Mol freigesetztes Chlor ca. 1 Mol Cyanamid verwendet wird. So beträgt beispielsweise das Molverhältnis Cyanamid : Siliciumtetrachlorid 1 : 0,25, wobei jedoch größere Überschüsse nicht kritisch sind.

Neben den genannten Synthesemöglichkeiten mit Cyanamid als Ausgangsverbindung eignen sich erfindungsgemäß auch Chlorformamidinium-Salze zur Erzeugung des Isoharnstoff-Salzes: Man setzt hierfür beispielsweise Chlorformamidinium-chlorid mit Ethanol um, wobei diese Umsetzung gemäß vorliegender Erfindung auch in Gegenwart von Cyanamid geschehen kann. Das Molverhältnis von Chlorformamidinium-chlorid zu Cyanamid beträgt etwa 1:1. Ethanol wird im Überschuß verwendet. Die Umsetzung erfolgt vorzugsweise im Temperaturbereich 10 bis 90°C. Anstelle von Chlorformamidinium-chlorid können auch andere Salze wie z.B. das Nitrat verwendet werden.

Der große technische und wirtschaftliche Vorteil der vorliegenden Erfindung besteht unter anderem darin, daß das O-Ethylisoharnstoff-Salz nicht isoliert und getrocknet werden muß. Das in situ hergestellte O-Ethylisoharnstoff-Salz kann direkt ohne vorhergehende Isolierung in der zweiten Reaktionsstufe umgesetzt werden. Das O-Ethylisoharnstoff-Salz kann in Form einer Lösung oder Suspension vorzugsweise in Ethanol eingesetzt werden. Weiterhin kann durch Zugabe von Alkoholat, insbesondere Alkalialkoholat, die O-Ethylisoharnstoffbase direkt aus dem Salz freigesetzt werden.

Gemäß dem Stand der Technik konnte nicht erwartet werden, daß in situ hergestellte O-Ethylisoharnstoff-Salze ohne Isolierung und Reinigung in Gegenwart aller Verunreinigungen zu reinem 2-Ethoxy-4,6-dihydroxypyrimidin umgesetzt werden können. Erfahrungsgemäß sind Heterocyclensynthesen sehr sensibel auf Verunreinigungen in den Ausgangsstoffen.

Im Verfahrensschritt b) wird anschließend der O-Ethylisoharnstoff oder dessen Salz mit einem Malonsäuredialkylester in Gegenwart eines Alkoholats, bei Temperaturen zwischen -10 und 180°C, bevorzugt zwischen 70 und 140°C, umgesetzt. Die Umsetzung erfolgt in Gegenwart von einem Lösungsmittel oder/und überschüssigem Malonsäuredialkylester. Als Lösungsmittel wird vorzugsweise ein Alkohol wie z.B. Ethanol oder ein Gemisch von Ethanol und Methanol verwendet, doch kann ohne weiteres auch auf andere Lösungsmittel wie z.B. Kohlenwasserstoffe oder Ether zurückgegriffen werden. Wenn das O-Ethylisoharnstoff-Salz als ethanolische Lösung oder Suspension und das Alkoholat als alkoholische Lösung eingesetzt werden, ist im allgemeinen die Zugabe einer weiteren Lösungsmittelmenge nicht erforderlich. Als Alkoholate werden vorzugsweise Alkalialkoholate, insbesondere Natrium- und Kaliumalkoholate von aliphatischen primären, sekundären und tertiären Alkoholen in fester Form oder als alkoholische Lösungen verwendet. Für die Herstellung von sehr reinem 2-Ethoxy-4,6-dihydropyrimidin wird bevorzugt Natriumethylat eingesetzt. In Gegenwart von Natriummethylat und Methanol wird 2-Methoxy-4,6-dihydroxypyrimidin als Nebenprodukt gebildet. Die Verwendung einer kostengünstigen technischen 30 %-igen methanolischen Natriummethylatlösung ist deshalb vor allem dann von Interesse, wenn es auf eine hohe Reinheit des Produktes nicht ankommt.

Als Malonsäuredialkylester werden aliphatische Malonsäureester insbesondere mit 1 bis 4 Kohlenstoffatomen, vorzugsweise Malonsäurediethylester, eingesetzt. Der Malonsäuredialkylester kann in stöchiometrischen Mengen bis zu einem großen Überschuß eingesetzt werden. Vorzugsweise werden pro 1 Mol eingesetztes Cyanamid bzw. pro 1 Mol O-Ethylisoharnstoff-Kation bzw. pro 1 Mol O-Ethylisoharnstoff-Base 1 bis 1,5 Mol Malonsäuredialkylester eingesetzt. Der Malonsäuredialkylester kann jedoch auch als Lösungsmittel verwendet werden.

Anstelle des Malonsäuredialkylesters können erfindungsgemäß auch dessen Salze, insbesondere Alkalisalze wie etwa Natrium- oder Kaliumsalz eingesetzt werden. Alkalisalze des Malonsäuredialkylesters sind in einfacher Weise, beispielsweise durch Umsetzung von Malonsäuredialkylester mit Alkalialkoholat, wie etwa Natriumethylat oder -methylat, zugänglich. Die Alkalisalze des Malonsäuredialkylesters können in situ hergestellt werden. In diesem Fall wird im Verfahrensschritt b) bevorzugt das erzeugte O-Ethylisoharnstoff-Salz mit einem Natrium-Malonsäuredialkylester, insbesondere Natrium-Malonsäurediethylester umgesetzt. Der Natrium-Malonsäuredialkylester fungiert bei dieser Variante sowohl als starke Base als auch als Malonsäuredialkylester-Quelle.

Überraschenderweise wurde gefunden, daß die vorliegende Erfindung die Verwendung von rohem, ungereinigtem in situ hergestelltem O-Ethylisoharnstoff ermöglicht. Der nicht isolierte, intermediär erzeugte O-Ethylisoharnstoff wird als Lösung in Ethanol oder in einem Gemisch von Ethanol und Methanol verwendet.

In einer bevorzugten Ausführungsform des erfindungsgemäßen Verfahrens beträgt das Molverhältnis von Isoharnstoff oder dessen Salz zum Alkoholat bzw. zum Salz des Malonsäuredialkylesters 1:1 bis 8. Bei Verwendung eines O-Ethylisoharnstoff-Salzes einer einwertigen Säure werden pro 1 Mol O-Ethylisoharnstoff-Salz im allgemeinen 1 bis 4 Mol Alkoholat, vorzugsweise 2 bis 2,5 Mol Alkoholat, eingesetzt. Bei Verwendung eines 0-Ethylisoharnstoff-Salzes einer zweiwertigen Säure werden pro 1 Mol O-Ethylisoharnstoff-Salz im allgemeinen 2 bis 8 Mol Alkoholat, vorzugsweise 4 bis 5 Mol Alkoholat, eingesetzt. Bei Verwendung von O-Ethylisoharnstoff-hydrogensulfat werden pro 1 Mol O-Ethylisoharnstoff-Salz im allgemeinen 2 bis 5 Mol Alkoholat, vorzugsweise 3 bis 4,5 Mol Alkoholat, eingesetzt. Bei Verwendung von O-Ethylisoharnstoff-Base ist die Gegenwart von Alkoholat nicht unbedingt erforderlich, zur Erzielung einer optimalen Ausbeute wird jedoch vorzugsweise pro 1 Mol O-Ethylisoharnstoff 1 bis 1,5 Mol Alkoholat eingesetzt.

Gemäß der vorliegenden Erfindung kann der Verfahrensschritt b) folgendermaßen durchgeführt werden:
intermediär hergestelltes, nicht isoliertes O-Ethylisoharnstoff-Salz oder freier O-Ethylisoharnstoff werden als ethanolische Lösung oder Suspension mit Alkoholat, vorzugsweise Natriumethylat, als feste Substanz oder alkoholische Lösung versetzt. Anschließend erfolgt die Zugabe des Malonsäuredialkylesters. Zur Vervollständigung der Umsetzung wird nachgerührt.

Es können aber auch Malonsäurediethylester und das O-Ethylisoharnstoff-Salz in Form einer ethanolischen Lösung oder einer Suspension oder der freie O-Ethylisoharnstoff vorgelegt werden. Bei dem O-Ethylisoharnstoff-Salz handeles sich um ein intermediär erzeugtes Produkt
Anschließend wird Alkoholat als Festprodukt oder alkoholische Lösung vorzugsweise bei -10 bis 150°C eingetragen. Die Reaktion wird durch Nachrühren vervollständigt.

Gemäß einer weiteren Aus führungs form kann auch in der Weise verfahren werden, daß der Malonsäuredialkylester als Lösungsmittel vorgelegt wird und ein alkoholisches Reaktionsgemisch von O-Ethylisoharnstoff-Salz und Alkalialkoholat bei 80 bis 180°C zugegeben wird. Anstelle der Reaktionsmischung von O-Ethylisoharnstoff-Salz und Alkoholat kann erfindungsgemäß auch eine alkoholische Mischung von O-Ethylisoharnstoff und Alkoholat verwendet werden, oder aber das O-Ethylisoharnstoff-Salz oder der O-Ethylisoharnstoff und das Alkoholat werden getrennt dem vorgelegten Malonsäuredialkylester zugegeben.

Überraschenderweise wurde gefunden, daß 2-Ethoxy-4,6-dihydroxypyrimidin auch noch bei hohen Temperaturen hergestellt werden kann, ohne daß die Reinheit und die Ausbeute stark vermindert werden. Dies konnte gemäß dem Stand der Technik, wonach O-Ethylisoharnstoff keine sehr stabile Verbindung ist, nicht erwartet werden, da O-Ethylisoharnstoff stark zu Zersetzung oder Trimerisierung neigt.

Gemäß dem Stand der Technik konnte nicht erwartet werden, daß Verfahrensschritt b) die Herstellung von 2-Ethoxy-4,6-dihydroxypyrimidin bzw. dessen Salz unter technisch gangbaren, wirtschaftlichen und ökologischen Bedingungen erlauben würde.

Im Verfahrensschritt c) kann dann das im Verfahrensschritt b) erhaltene Salz des 2-Ethoxy-4,6-dihydroxypyrimidins (insbesondere das Natrium- oder Kaliumsalz) durch eine Säure in das gewünschte 2-Ethoxy-4,6-dihydroxypyrimidin übergeführt werden. Dazu wird im Anschluß an Verfahrensstufe b) das Reaktionsgemisch soweit eingeengt, daß ein noch rührbares Gemisch vorliegt. Nach Zugabe von Wasser wird ein pH-Wert von 2,0 bis 9,0, vorzugsweise 3,5 bis 5,5, eingestellt. Man kann auch so verfahren, daß während des Abdestillierens des Alkohols bzw. des Lösungsmittels Wasser zugesetzt wird.

In einer weiteren Ausführungsform wird das Pyrimidinsalz, gegebenenfalls zusammen mit dem Alkalisalz der eingesetzten Säure, in fester Form isoliert. Dies kann durch Filtration geschehen, wobei gegebenenfalls das Reaktionsgemisch vorher noch eingedampft werden muß, oder durch Eindampfen des Reaktionsgemisches zur Trockene in einem Trockner oder Dünnschichtverdampfer. Das isolierte Pyrimidinsalz wird in Wasser gelöst oder suspendiert und anschließend angesäuert. Das Ansäuern erfolgt mit einer Mineralsäure oder einer Carbonsäure, z.B. Essigsäure. Das 2-Ethoxy-4,6-dihydroxypyrimidin fällt in kristalliner Form an.

Das auf diese Weise als Feststoff anfallende 2-Ethoxy-4,6-dihydroxypyrimidin wird gegebenenfalls in einem anschließenden Aufarbeitungsschritt mit Wasser salzfrei gewaschen und abschließend gegebenenfalls getrocknet.

Das erfindungsgemäße Verfahren erlaubt die Synthese von 2-Ethoxy-4,6-dihydroxypyrimidin ohne die Isolierung intermediär gebildeter Produkte in guten Ausbeuten und hoher Reinheit.

In den Beispielen 1 bis 5, 10 und 15 soll die Erfindung weiter veranschaulicht werden. Die Beispiele 6 bis 9 und 11 bis 14 stellen aufgrund der Verwendung von nicht in situ erzeugtem O-Ethylisoharnstoff keine Beispiele der Erfindung dar.

### Beispiele

### Beispiel 1

### Ausgangsverbindungen: Cyanamid und Chlorwasserstoff in Ethanol

In 75 g Ethanol wurden während 150 Minuten bei 15 bis 20°C 9,5 g (0,26 Mol) trockener Chlorwasserstoff eingeleitet. Anschließend wurden bei 20°C 10,56 g (0,25 Mol) Cyanamid innerhalb von 15 Minuten zugegeben. Nach 24 Stunden, als das Cyanamid vollständig umgesetzt war, wurde die Lösung auf -10°C gekühlt; dann wurden ebenfalls bei -10°C - 162,02 g (0,5 Mol) einer 21 %-igen Lösung von Natriumethylat in Ethanol während 60 Minuten zugetropft. Nach der Zudosierung von 44,05 g (0,28 Mol) Malonsäurediethylester während 15 Minuten wurde 7 Tage bei Raumtemperatur gerührt und anschließend im Vakuum bis zur Trockene eingedampft. Die Lösung des mit 280 g Wasser aufgenommenen Rückstandes wies einen pH-Wert von 10,3 auf. Diese Lösung wurde in eine Mischung aus 30 g (0,5 Mol) Essigsäure und 200 g Wasser eingerührt, das auskristallisierte Produkt abfiltriert, mit 100 ml Wasser gewaschen und im Vakuum bei 75°C getrocknet.
- Ausbeute:: 28,1 g = 71,9 %
- Gehalt (acidimetrisch):: 99,4 %
- Schmelzpunkt:: > 360°C (ab 200°C beginnende Zersetzung)

Stellvertretend für die nachfolgenden Beispiele werden an dieser Stelle die repräsentativen Ergebnisse der Elementaranalyse sowie des NMR-Spektrums des Produktes aus Beispiel 1 angeführt:

Elementaranalyse: Zur Gewinnung von Reinsubstanz wurde die Substanz aus Ethanol/Wasser umkristallisiert und über P₂O₅ im Vakuumexsiccator getrocknet.

| | Analyse [%] | Theorie [%] |
|---|---|---|
| C | 45,91 | 46,15 |
| H | 5,10 | 5,17 |
| N | 17,94 | 17,94 |

¹H-NMR-Spektrum ([D₆]DMSO):
FT-NMR-Spektrometer
Typ: Joel-JMX-GX 400
Frequenz: 100,5 MHz (C)
δ (ppm) = 1,17 bis 1,29 (t; 3H, J = 7H₂, 2-CH₃-CH₂O-) ;
4,25 bis 4,33 (q; 2H, J = 7H₂, 2-CH₃-CH₂O-);
4,95 (s; 1H, -CH-);
11,47 (S_{breit}; 2H, -OH, -OH);

### Beispiel 2

### Ausgangsverbindungen: Cyanamid und Schwefelsäure in Ethanol

15 kg wasserfreies Ethanol wurden unter N₂-Deckung vorgelegt und auf -5 bis -10°C abgekühlt. Dann wurde während ca. 15 Minuten 2,5 kg (25 Mol) 96 %-ige Schwefelsäure unter Rühren bei -5°C zudosiert. Bei 0 bis +10°C wurden anschließend insgesamt 1,05 kg (25 Mol) Cyanamid portionsweise und über 60 Minuten eingerührt. Bei 20°C beträgt die Nachreaktionszeit zum vollständigen Abreagieren des Cyanamids etwa 6 Stunden.

Nach Abkühlen der erhaltenen Lösung von O-Ethylisoharnstoff-hydrogensulfat auf -10°C wurden über 60 Minuten 25,5 kg (75 Mol) einer 20 %-igen Lösung von Natriumethylat in Ethanol zudosiert und nach weiteren 5 Minuten Nachreaktion, wiederum bei -10°C, 4,4 kg (27,5 Mol) Malonsäurediethylester innerhalb ca. 15 Minuten zugetropft. Das Reaktionsgemisch wurde 90 Stunden lang bei 25°C gerührt; dann wurden mehr als 2/3 des Ethanols abdestilliert. Anschließend wurde zum Lösen des Destillationsrückstandes Wasser zugegeben. Der pH-Wert nach dem Lösen des Feststoffes betrug 8,0 bis 11,0.

Mit 3,6 kg 18 %-iger Salzsäure wurde bei 10 bis 15°C der pH auf einen Endwert von 4,0 eingestellt. Nach dem Abtrennen des auskristallisierten Produktes und Sulfatfreiwaschen mit 7,5 kg Wasser wurde bei 75°C im Vakuum getrocknet.
- Ausbeute:: 2,1 kg = 57 %
- Gehalt: (HPLC):: 99 %

### Beispiel 3

### Ausgangsverbindungen: Cyanamid und Methansulfonsäure in Ethanol

Zu 250 g Ethanol wurden innerhalb von 5 Minuten bei 22°C und unter leichter Kühlung 48,5 g (0,5 Mol) Methansulfonsäure gegeben. 21,1 g (0,5 Mol) Cyanamid, die in 100 g Ethanol gelöst worden waren, wurden bei 20 bis 25°C während 30 Minuten zugegeben. Nach 17 Stunden Rühren bei Raumtemperatur war das Cyanamid vollständig umgesetzt. 209,35 g (0,25 Mol) der Lösung wurden auf -10°C gekühlt und dieser Lösung anschließend während 60 Minuten 236,3 g (0,75 Mol) einer 21,6 %-igen Lösung von Natriumethylat in Ethanol zugegeben.

Nach 5 minütigem Rühren wurden während 15 Minuten bei -10°C 44,05 g (0,275 Mol) Malonsäurediethylester zugetropft. Für die sich anschließende Nachreaktion wurde das Gemisch zunächst für 4 Stunden bei 0°C und anschließend 3 Tage lang bei Raumtemperatur gehalten.

Der Reaktionsansatz wurde im Vakuum eingedampft, der Rückstand mit 560 g Wasser bei 18°C gelöst und der pH-Wert anschließend mit 6,3 g 32 %-iger Salzsäure auf 4,0 eingestellt. Das nach einigen Minuten auskristallisierende Produkt wurde abfiltriert, mit 100 g Wasser salzfrei gewaschen und im Vakuum bei 80°C getrocknet.
- Ausbeute:: 19,75 g = 50,6 %
- Gehalt (acidimetrisch):: 99,5 %

### Beispiel 4

### Ausgangsverbindungen: Cyanamid und Siliciumtetrachlorid

4,25 g (0,025 Mol) Siliciumtetrachlorid wurden in 46 g (1 Mol) Ethanol bei ca. 20°C eingerührt. Daraufhin wurden während 10 Minuten bei ca. 12°C 4,22 g (0,1 Mol) 99,5 %-iges Cyanamid zugesetzt, die nach 20 Stunden vollständig umgesetzt waren. Der auf -5°C gekühlten farblosen Lösung wurden innerhalb von 10 Minuten 64,8 g (0,2 Mol) einer 21 %-igen Lösung von Natriumethylat in Ethanol und während 5 Minuten 17,62 g (0,11 Mol) Malonsäurediethylester zugetropft.

Nach 18 Tagen Rühren bei Raumtemperatur wurde bei 40°C eingedampft. Die kolloidalen Anteile des mit Wasser aufgenommenen Niederschlages wurden abfiltriert und das Filtrat mit 11,6 g 32 %-iger Salzsäure auf pH 4,0 eingestellt; anschließend wurde das durch Ansäuern ausgefällte Produkt durch Filtrieren abgetrennt, mit Wasser gewaschen und bei 75°C getrocknet.
- Ausbeute:: 13,2 g = 84,2 %
- Gehalt (acidimetrisch):: 100 %

### Beispiel 5

### Ausgangsverbindungen: Cyanamid und Thionylchlorid

Eine Lösung von 4,22 g (0,1 Mol) 99,5 %-igem Cyanamid in 50 g Ethanol wurde unter Rühren und Kühlen mit 5,95 g (0,05 Mol) Thionylchlorid langsam versetzt. Die exotherm verlaufende Bildung des O-Ethylisoharnstoff-hydrochlorids war nach 21 Stunden bei 21°C quantitativ abgelaufen. Nach Abfiltrieren wurden zu dem Filtrat bei etwa 10°C und innerhalb von 30 Minuten 102,7 g (0,3 Mol) einer 20 %-igen Lösung von Natriumethylat in Ethanol und 16,02 g (0,1 Mol) Malonsäurediethylester gegeben. 90 Minuten lang wurde Ethanol bei 80°C destillativ abgetrennt und anschließend der feste Rückstand in 205 ml Wasser gelöst. Die alkalische Lösung (pH 12,4) wurde mit 18,4 g 32 %-iger Salzsäure (0,16 Mol) auf pH 4,0 eingestellt; nach kurzem Kühlen im Eisbad wurde der Niederschlag abgesaugt, gewaschen und bei 75°C getrocknet.
- Ausbeute:: 8,49 g = 54,4 %
- Gehalt (acidimetrisch):: 98,4 %

### Beispiel 6

### Ausgangsverbindung: O-Ethylisoharnstoff-hydrogensulfat

Bei -10°C wurden 490 g (2,5 Mol) 95 %-iges O-Ethylisoharnstoff-hydrogensulfat in 1500 g wasserfreies Ethanol eingetragen und anschließend während 45 Minuten 2430 g (7,5 Mol) einer 21 %-igen Lösung von Natriumethylat in Ethanol unter Rühren zugetropft. Nach 5 Minuten Nachreaktionszeit wurden innerhalb weiterer 15 Minuten 440,5 g (2,75 Mol) Malonsäurediethylester bei -10°C zugetropft und das Gemisch anschließend 4 Stunden lang unter Rückfluß erhitzt. Dann wurden etwa 2/3 des Alkohols abdestilliert und durch die entsprechende Wassermenge ersetzt, ohne daß der Destillationsvorgang dadurch unterbrochen wurde. Insgesamt wurden 4,2 kg wäßrig-alkoholisches Destillat erhalten. Die Mischung wurde mit ca. 6 kg Wasser versetzt und auf 15°C abgekühlt. Das Produkt wurde mit 196 g (1,72 Mol) 32 %-iger Salzsäure ausgefällt, abgesaugt, mit Wasser gewaschen und bei 75°C getrocknet.
- Ausbeute:: 212 g = 54 %
- Gehalt (HPLC):: 99 %

### Beispiel 7

### Ausgangsverbindung: O-Ethylisoharnstoff-hydrochlorid

62,3 g (0,5 Mol) O-Ethylisoharnstoff-hydrochlorid wurden in 150 g Ethanol eingetragen, das bei -5°C vorgelegt worden war. Dann wurden während 45 Minuten 324 g (1,0 Mol) einer 21 %-igen Lösung von Natriumethylat in Ethanol und anschließend während 10 Minuten 88,1 g (0,5 Mol) Malonsäurediethylester zugetropft. Das Reaktionsgemisch wurde über 4 Stunden bei 0°C und anschließend 90 Stunden bei Raumtemperatur gerührt. Nach dem Eindampfen wurde der Rückstand in eine Lösung aus 30,4 g (0,5 Mol) Eisessig in 300 g Wasser gerührt und das Produkt abgetrennt; dieses wurde nach dem Abkühlen auf 17°C (pH 6,2) gewaschen und bei 75°C getrocknet. Zur Mutterlauge wurden nochmals 15,2 g (0,25 Mol) Essigsäure gegeben und diese wie oben aufgearbeitet.
- Gesamtausbeute:: 52,8 g = 68 %
- Gehalt (HPLC):: 95,9 %

### Beispiel 8

### Ausgangsverbindungen: Chlorformamidinium-chlorid und Ethanol

11,5 g (0,1 Mol) Chlorformamidinium-chlorid wurden zu 30 g Ethanol (99 %-ig) gegeben und unter Wasserausschluß unter Rückfluß gekocht. Nach 30 Minuten war das Salz gelöst und nach 120 Minuten das Chlorformamidinium-chlorid quantitativ zu 0-Ethylisoharnstoff-hydrochlorid umgesetzt. Nachdem die Lösung im Vakuum zur Trockne eingedampft worden war, wurde der feste, weiße Rückstand in 30 g Ethanol gelöst und zu dieser Lösung bei 25 °C 63 g (0,2 Mol) einer 21,6 %-igen Lösung von Natriumethylat in Ethanol über 15 Minuten zugetropft. Anschließend wurden 16,02 g (0,1 Mol) Malonsäurediethylester zugegeben und das Gemisch 17 Stunden lang bei 25°C gerührt. Nachdem weitere 60 Minuten unter Rückfluß gekocht und dann im Vakuum eingedampft worden war, wurde der feste Rückstand in 150 g Wasser gelöst und der pH-Wert der Lösung mit verdünnter Salzsäure auf 4,0 eingestellt. Das kristalline Fällungsprodukt wurde abgesaugt, mit Wasser salzfrei gewaschen und bei 75°C im Vakuum getrocknet.
- Ausbeute:: 6,4 g = 42,7 %
- Gehalt (acidimetrisch):: 99,5 %

### Beispiel 9

### Ausgangsverbindungen: Chlorformamidinium-chlorid und Cyanamid in Ethanol

11,5 g (0,1 Mol) Chlorformamidinium-chlorid wurden zusammen mit 30 g Ethanol (99 %-ig) und 4,2 g (0,1 Mol) Cyanamid bei 25°C unter Wasserausschluß gerührt. Die Komponenten befanden sich nach 120 Minuten in der gelösten Form und hatten sich nach insgesamt 20 Stunden quantitativ zum O-Ethylisoharnstoff-hydrochlorid umgesetzt. Anschließend wurde die Lösung im Vakuum eingedampft und 12,4 g (0,1 Mol) des festen Salzes in Ethanol wieder gelöst. Unter Kühlen im Eisbad wurden 63 g (0,2 Mol) einer 21,6 %-igen Lösung von Natriumethylat in Ethanol bei 0°C während 15 Minuten zugetropft, dann 16,02 g (0,1 Mol) Malonsäurediethylester zugegeben und das Reaktionsgemisch 18 Stunden bei 25°C gerührt. Nach 1 Stunde Kochen unter Rückfluß wurde im Vakuum eingedampft, der Rückstand anschließend in 150 g Wasser gelöst und der pH-Wert mit verdünnter Salzsäure auf 3,0 bis 4,0 eingestellt. Der kristalline Niederschlag wurde abfiltriert, das Produkt mit Wasser chloridfrei gewaschen und bei 75°C im Vakuum getrocknet.
- Ausbeute:: 6,4 g = 41,0 %
- Gehalt (acidimetrisch):: 98,7 %

### Beispiel 10

### Ausgangsverbindungen: Chlorformamidinium-chlorid und Cyanamid in Ethanol

Entsprechend Beispiel 9 wurde eine Lösung von 0,25 Mol O-Ethylisoharnstoff-hydrochlorid in Ethanol aus Chlorformamidinium-chlorid und Cyanamid hergestellt und anschließend mit 0,5 Mol einer 21 %-igen Natriummethylatlösung in Ethanol bei 10°C versetzt. Das so erhaltene Reaktionsgemisch wurde in 140 g (0,87 Mol) Malonsäurediethylester bei 130 bis 145°C unter Rühren während 3 Stunden eingetropft und das Ethanol im schwachen Stickstoffstrom abdestilliert. Nach Abkühlen auf 15°C wurde der feste aus Natrium-2-ethoxy-4,6-dihydroxypyrimidin und Natriumchlorid bestehende Rückstand abgesaugt, mit Ethanol gewaschen und anschließend entsprechend Beispiel 9 aufgearbeitet.
- Ausbeute:: 28,1 g = 72,0 %
- Gehalt (HPLC):: 99,3 %

### Beispiel 11

### Ausgangsverbindungen: O-Ethylisoharnstoff-hydrogensulfat in Methanol und Natriummethylat

Zu 98 g (0,5 Mol) O-Ethylisoharnstoff-hydrogensulfat in 300 g wasserfreiem Methanol wurden bei -1 bis -7°C 270 g (1,5 Mol) einer 30 %-igen Lösung von Natriummethylat in Methanol während 75 Minuten getropft. Dann wurden bei -5 °C und innerhalb von 30 Minuten 66,1 g (0,5 Mol) Malonsäuredimethylester zudosiert und das Reaktionsgemisch 3 Stunden bei 0°C und anschließend 5 Tage bei 25°C gerührt. Nach mildem Eindampfen bei 30 bis 40°C im Vakuum wurden 188,6 g eines weißlich-gelben Rückstandes erhalten, der mit 1490 g Wasser in eine gelbe Lösung übergeführt wurde (pH: 10,0). Mit 54,2 g (0,475 Mol) einer 32 %-igen Salzsäure wurde ein weißer Niederschlag ausgefällt, abfiltriert, mit 250 g Wasser gewaschen und das Produkt bei 80°C im Vakuum getrocknet.
- Ausbeute:: 54,3 g = 69,5 %
- Gehalt (HPLC):: 91,4 % 2-Ethoxy-4,6-dihydroxypyrimidin
8,4 % 2-Methoxy-4,6-dihydroxypyrimidin

Das NMR-Spektrum dieses Produktes weist zusätzlich zu den bekannten Peaks (vgl. Beispiel 1) einen Peak bei 3,82 ppm (-OCH₃) auf.

### Beispiel 12

### Ausgangsverbindungen: O-Ethylisoharnstoff und Natriumethylat (Molverhältnis 1:1)

18,6 g (0,2 Mol) O-Ethylisoharnstoff (Gehalt: 94,8 %) wurden innerhalb 3 Minuten bei 20°C zu 32,04 g (0,2 Mol) Malonsäurediethylester gegeben. Anschließend wurden über 35 Minuten bei 12 bis 25°C 65,7 g (0,2 Mol) einer 20,7 %-igen Lösung von Natriumethylat in Ethanol zudosiert, das Reaktionsgemisch 70 Stunden bei 20°C gerührt und dann bei 50°C im Vakuum eingedampft. Man erhielt 40,4 g eines gelben Feststoffes, der in 400 g Wasser gelöst wurde. Die Lösung wurde auf 10 bis 15°C abgekühlt und der pH-Wert durch Zugabe von 24,2 g (0,21 Mol) einer 32 %-igen Salzsäure von 11,0 auf 4,0 gebracht. Nach 30 Minuten Rühren im Eisbad wurde abfiltriert, das Produkt mit Wasser gewaschen und bei 75°C im Vakuum getrocknet.
- Ausbeute:: 18,61 g = 59,6 %
- Gehalt (acidimetrisch):: 98,0 %
(HPLC): 97,5 %

### Beispiel 13

### Ausgangsverbindung: O-Ethylisoharnstoff (Molverhältnis Base : Ester = 2:1)

18,6 g (0,2 Mol) der 94,8 %-igen O-Ethylisoharnstoffbase wurden 70 Stunden bei Raumtemperatur in 16,02 g (0,1 Mol) Malonsäurediethylester eingerührt. Die fast feste Mase wurde anschließend bei 50°C im Vakuum vom gebildeten Ethanol befreit. Die 45 g des Rückstandes fettiger Konsistenz wurden in 500 g Wasser gelöst und der pH-Wert anschließend mit 4,1 g konzentrierter Schwefelsäure auf 3,4 eingestellt. Nach 15 Minuten Rühren im Eisbad wurde abfiltriert, das Produkt mit Wasser gewaschen und bei 75°C im Vakuum getrocknet.
- Ausbeute:: 5,06 g = 32,4%
- Gehalt (acidimetrisch):: 97,4 %

### Beispiel 14

### Ausgangsverbindungen: O-Ethylisoharnstoff-hydrogensulfat Natriumsalz des Malonsäurediethylesters

3400 g wasserfreies Ethanol und 1367 g (7,5 Mol) Natrium-Malonsäurediethylester wurden vorgelegt. Anschließend wurden bei -5 bis 0°C 466 g (2,5 Mol) reines O-Ethylisoharnstoff-hydrogensulfat portionsweise unter kräftigem Rühren eingetragen. Anschließend wurde 24 Stunden lang unter Rückfluß erhitzt. Dann wurde das Reaktionsgemisch gemäß Beispiel 6 aufgearbeitet.
- Ausbeute:: 285 g = 73 %
- Gehalt (HPLC):: 99,2 %

### Beispiel 15

### Ausgangsverbindung: O-Ethylisoharnstoff-hydrochlorid in ethanolischer Lösung

Gemäß Beispiel 1 wurde eine Lösung von 0,5 Mol O-Ethylisoharnstoff-hydrochlorid in 150 g Ethanol (absolut) hergestellt. Anschließend wurde 1 Mol einer 21 %-igen ethanolischen Natriumethylatlösung bei 0°C unter Rühren zugegeben. Das ausgefallene Natriumchlorid wurde unter Ausschluß von Luftfeuchtigkeit abfiltriert und das Filtrat bei 20°C mit 88,5 g (0,55 Mol) Malonsäurediethylester während 1 Stunde versetzt. Das Reaktionsgemisch wurde anschließend 25 Stunden unter Rückfluß erhitzt und danach am Rotationsverdampfer so weit eingeengt, daß gerade noch eine rühr- und pumpbare Suspension vorlag. Es wurde der Niederschlag abgesaugt, mit Ethanol gewaschen und im Vakuum einer Wasserstrahlpumpe bei 90°C getrocknet.
- Ausbeute:: 69 g Natriumsalz des 2-Ethoxy-4,6-dihydroxypyrimidins
= 77,5 %
- Schmelzpunkt:: > 360°C
- Gehalt (HPLC):: 99 %

## Patentansprüche

1. Verfahren zur Herstellung von 2-Ethoxy-4,6-dihydroxypyrimidin oder dessen Salz durch Umsetzung von O-Ethylisoharnstoff oder dessen Salz mit einem Malonsäuredialkylester sowie gegebenenfalls Überführung des erhaltenen Salzes des 2-Ethoxy-4,6-dihydroxypyrimidins durch eine Säure in das freie Pyrimidin-Derivat,
**dadurch gekennzeichnet,**
dass man
a) den O-Ethylisoharnstoff oder dessen Salz aus Cyanamid oder Chlorformamidiniumsalzen intermediäre erzeugt und
b) anschließend den Isoharnstoff oder dessen Salz in situ mit dem Malonsäuredialkylester und einem Alkoholat oder einem Salz des Malonsäuredialkylesters in Gegenwart eines Lösungsmittels von -10 bis 180 °C reagieren lässt.

2. Verfahren nach Anspruch 1,
**dadurch gekennzeichnet,**
daß man als Alkoholat Alkalialkoholat, insbesondere Natriumalkoholat verwendet.

3. Verfahren nach Anspruch 1 oder 2,
**dadurch gekennzeichnet,**
daß man das O-Ethylisoharnstoffsalz durch Umsetzung von Cyanamid mit Ethanol in Gegenwart einer Mineralsäure, wasserfreiem Chlorwasserstoff oder einer starken organischen Säure erzeugt.

4. Verfahren nach Anspruch 3,
**dadurch gekennzeichnet,**
daß man als Mineralsäure konzentrierte Schwefelsäure (> 95 %) oder eine Mischung aus technischer Schwefelsäure und rauchender Schwefelsäure einsetzt.

5. Verfahren nach Anspruch 3,
**dadurch gekennzeichnet,**
daß man als starke organische Säure eine Sulfonsäure oder eine Halogenessigsäure verwendet.

6. Verfahren nach Anspruch 5,
**dadurch gekennzeichnet,**
daß man Methansulfonsäure, Benzolsulfonsäure, p-Toluolsulfonsäure oder Trichloressigsäure verwendet.

7. Verfahren nach Anspruch 1 oder 2,
**dadurch gekennzeichnet,**
daß man das O-Ethylisoharnstoffsalz durch Umsetzung von Cyanamid mit Ethanol in Gegenwart einer HCl-abspaltenden Verbindung erzeugt.

8. Verfahren nach Anspruch 7,
**dadurch gekennzeichnet,**
daß man als HCl-abspaltende Verbindung Siliciumtetrachlorid oder Thionylchlorid einsetzt.

9. Verfahren nach Anspruch 1 oder 2,
**dadurch gekennzeichnet,**
daß man das O-Ethylisoharnstoffsalz durch Umsetzung von Chlorformamidinumchlorid mit Ethanol erzeugt.

10. Verfahren nach Anspruch 1 oder 2,
**dadurch gekennzeichnet,**
daß man das O-Ethylisoharnstoffsalz durch Umsetzung von Chlorformamidiniumchlorid mit Ethanol in Gegenwart von Cyanamid erzeugt.

11. Verfahren nach einem der vorhergehenden Ansprüche,
**dadurch gekennzeichnet,**
daß man das O-Ethylisoharnstoffsalz bei Temperaturen zwischen -10 und +90 °C erzeugt.

12. Verfahren nach einem der vorhergehenden Ansprüche,
**dadurch gekennzeichnet,**
daß im Verfahrensschritt (b) das Molverhältnis von Isoharnstoff oder dessen Salz zum Alkoholat bzw. zum Salz des Malonsäuredialkylesters 1:1 bis 8 beträgt.

13. Verfahren nach einem der vorhergehenden Ansprüche,
**dadurch gekennzeichnet,**
daß im Verfahrensschritt (b) als Alkoholat Natriumethylat eingesetzt wird.

14. Verfahren nach einem der vorhergehenden Ansprüche,
**dadurch gekennzeichnet,**
daß man den Verfahrensschritt (b) im Temperaturbereich von 70 bis 140 °C durchführt.

15. Verfahren nach einem der vorhergehenden Ansprüche,
**dadurch gekennzeichnet,**
daß im Verfahrensschritt (b) ein O-Ethylisoharnstoffsalz mit einem Alkali-Malonsäuredialkylester, insbesondere Natrium-Malonsäuredialkylester umgesetzt wird.

16. Verfahren nach einem der vorhergehenden Ansprüche,
**dadurch gekennzeichnet,**
daß im Verfahrensschritt (b) Ethanol oder/und überschüssiger Malonsäuredialkylester als Lösungsmittel verwendet wird.

17. Verfahren nach einem der vorhergehenden Ansprüche,
**dadurch gekennzeichnet,**
daß als Malonsäuredialkylester Malonsäurediethylester verwendet wird.

18. Verfahren nach einem der vorhergehenden Ansprüche,
**dadurch gekennzeichnet,**
daß man nach Verfahrensschritt (b) das Lösungsmittel zumindest teilweise entfernt.

19. Verfahren nach Anspruch 18,
**dadurch gekennzeichnet,**
daß das Lösungsmittel durch Destillation entfernt wird.

20. Verfahren nach einem der vorhergehenden Ansprüche,
**dadurch gekennzeichnet,**
daß das Reaktionsprodukt aus Verfahrensschritt (b) in wäßriger Phase aufgenommen und auf einen pH-Wert zwischen 2,0 und 9,0 angesauert wird.

21. Verfahren nach Anspruch 20,
**dadurch gekennzeichnet,**
daß auf einen pH-Wert von 3,5 bis 5,5 angesäuert wird.

22. Verfahren nach Anspruch 21,
**dadurch gekennzeichnet,**
daß zum Ansäuern eine Mineral- oder Carbonsäure verwendet wird.

23. Verfahren nach Anspruch 22,
**dadurch gekennzeichnet,**
daß Salzsäure, Schwefelsäure oder Essigsäure verwendet wird.

24. Verfahren nach einem der Ansprüche 20 bis 23,
**dadurch gekennzeichnet,**
daß man in einem sich anschließenden Aufarbeitungsschritt das Produkt als Feststoff mit Wasser salzfrei wäscht und gegebenenfalls trocknet.

## Claims

1. Process for producing 2-ethoxy-4,6-dihydroxypyrimidine or the salt thereof by reacting O-ethylisourea or the salt thereof with a malonic acid dialkyl ester and optionally converting the salt of the 2-ethoxy-4,6-dihydroxypyrimidine obtained, by an acid, into the free pyrimidine derivative, characterised in that
a) the O-ethylisourea or the salt thereof is produced intermediately from cyanamide or chloroformamidinium salts and
b) the isourea or the salt thereof is then reacted in situ with the malonic acid dialkyl ester and an alkoxide or a salt of the malonic acid dialkyl ester from -10 to 180°C in the presence of a solvent.

2. Process according to claim 1, characterised in that an alkali metal alkoxide, in particular sodium alcoholate, is used as the alkoxide.

3. Process according to claim 1 or 2, characterised in that the O-ethylisourea salt is produced by reacting cyanamide with ethanol in the presence of a mineral acid, anhydrous hydrogen chloride or a strong organic acid.

4. Process according to claim 3, characterised in that concentrated sulphuric acid (> 95%) or a mixture of industrial sulphuric acid and fuming sulphuric acid is used as the mineral acid.

5. Process according to claim 3, characterised in that a sulphonic acid or a haloacetic acid is used as the strong organic acid.

6. Process according to claim 5, characterised in that methanesulphonic acid, benzenesulphonic acid, p-toluenesulphonic acid or trichloroacetic acid is used.

7. Process according to claim 1 or 2, characterised in that the O-ethylisourea salt is produced by reacting cyanamide with ethanol in the presence of a compound that cleaves off HCl.

8. Process according to claim 7, characterised in that silicon tetrachloride or thionyl chloride is used as the compound that cleaves off HCl.

9. Process according to claim 1 or 2, characterised in that the O-ethylisourea salt is produced by reacting chloroformamidinium chloride with ethanol.

10. Process according to claim 1 or 2, characterised in that the O-ethylisourea salt is produced by reacting chloroformamidinium chloride with ethanol in the presence of cyanamide.

11. Process according to any one of the preceding claims, characterised in that the O-ethylisourea salt is produced at temperatures between -10 and +90°C.

12. Process according to any one of the preceding claims, characterised in that in process stage (b), the mole ratio of isourea or its salt to the alkoxide or to the salt of the malonic acid dialkyl ester is 1:1 to 8.

13. Process according to any one of the preceding claims, characterised in that in process stage (b), sodium ethoxide is used as the alkoxide.

14. Process according to any one of the preceding claims, characterised in that process stage (b) is carried out in the temperature range 70 to 140°C.

15. Process according to any one of the preceding claims, characterised in that in process stage (b), an O-ethylisourea salt is reacted with an alkali malonic acid dialkyl ester, in particular sodium malonic acid dialkyl ester.

16. Process according to any one of the preceding claims, characterised in that in process stage (b), ethanol or/and excess malonic acid dialkyl ester is used as the solvent.

17. Process according to any one of the preceding claims, characterised in that malonic acid diethyl ester is used as the malonic acid dialkyl ester.

18. Process according to any one of the preceding claims, characterised in that, the solvent is at least partially removed after process stage (b).

19. Process according to claim 18, characterised in that the solvent is removed by distillation.

20. Process according to any one of the preceding claims, characterised in that the reaction product from process stage (b) is taken up in an aqueous phase and acidified to a pH value between 2.0 and 9.0.

21. Process according to claim 20, characterised in that it is acidified to a pH value of 3.5 to 5.5.

22. Process according to claim 21, characterised in that a mineral or carboxylic acid is used for the acidification.

23. Process according to claim 22, characterised in that hydrochloric acid, sulphuric acid or acetic acid is used.

24. Process according to any one of claims 20 to 23, characterised in that in a subsequent processing step the product in the form of a solid substance is washed salt-free with water and, if desired, dried.

## Revendications

1. Procédé de préparation de 2-éthoxy-4,6-dihydroxypyrimidine ou de son sel par réaction de la 0-éthylisourée ou de son sel avec un malonate de dialkyle et éventuellement conversion du sel de 2-éthoxy-4,6-dihydroxypyrimidine obtenu par un acide en le dérivé de pyrimidine libre,
caractérisé en ce que
a) on produit de manière intermédiaire la O-éthylisourée ou son sel à partir de cyanamide ou de sels de chloroformamidinium et
b) on fait ensuite réagir l'isourée ou son sel in situ avec le malonate de dialkyle et un alcoolate ou un sel du malonate de dialkyle en présence d'un solvant de -10 à 180°C.

2. Procédé selon la revendication 1 caractérisé en ce que l'on utilise comme alcoolate un alcoolate alcalin, en particulier un alcoolate de sodium.

3. Procédé selon la revendication 1 ou 2 caractérisé en ce que l'on produit le sel de 0-éthylisourée par réaction du cyanamide avec l'éthanol en présence d'un acide minéral, de chlorure d'hydrogène anhydre ou d'un acide organique fort.

4. Procédé selon la revendication 3 caractérisé en ce que l'on utilise comme acide minéral de l'acide sulfurique concentré (> 95 %) ou un mélange d'acide sulfurique technique et d'acide sulfurique fumant.

5. Procédé selon la revendication 3 caractérisé en ce que l'on utilise comme acide organique fort un acide sulfonique ou un acide halogénoacétique.

6. Procédé selon la revendication 5 caractérisé en ce que l'on utilise l'acide méthanesulfonique, l'acide benzènesulfonique, l'acide p-toluènesulfonique ou l'acide trichloroacétique.

7. Procédé selon la revendication 1 ou 2 caractérisé en ce que l'on produit le sel de O-éthylisourée par réaction du cyanamide avec l'éthanol en présence d'un composé produisant HCl.

8. Procédé selon la revendication 7 caractérisé en ce que l'on utilise comme composé produisant HCl le tétrachlorure de silicium ou le chlorure de thionyle.

9. Procédé selon la revendication 1 ou 2 caractérisé en ce que l'on produit le sel de 0-éthylisourée par réaction du chlorure de chloroformamidinium avec l'éthanol.

10. Procédé selon la revendication 1 ou 2 caractérisé en ce que l'on produit le sel de O-éthylisourée par réaction du chlorure de chloroformamidinium avec l'éthanol en présence de cyanamide.

11. Procédé selon l'une des revendications précédentes caractérisé en ce que l'on produit le sel de O-éthylisourée à des températures entre -10 et +90°C.

12. Procédé selon l'une des revendications précédentes caractérisé en ce que, dans l'étape de procédé (b), le rapport molaire de l'isourée ou de son sel à l'alcoolate ou au sel du malonate de dialkyle est de 1:1 à 8.

13. Procédé selon l'une des revendications précédentes caractérisé en ce que, dans l'étape de procédé (b), on utilise l'éthylate de sodium comme alcoolate.

14. Procédé selon l'une des revendications précédentes caractérisé en ce que l'on réalise l'étape de procédé (b) dans le domaine de température de 70 à 140°C.

15. Procédé selon l'une des revendications précédentes caractérisé en ce que, dans l'étape de procédé (b), un sel de O-éthylisourée est mis à réagir avec un malonate de dialkyle alcalin, en particulier un malonate de dialkyle sodique.

16. Procédé selon l'une des revendications précédentes caractérisé en ce que, dans l'étape de procédé (b), l'éthanol et/ou du malonate de dialkyle en excès est utilisé comme solvant.

17. Procédé selon l'une des revendications précédentes caractérisé en ce que le malonate de diéthyle est utilisé comme malonate de dialkyle.

18. Procédé selon l'une des revendications précédentes caractérisé en ce que l'on élimine le solvant au moins en partie après l'étape de procédé (b).

19. Procédé selon la revendication 18 caractérisé en ce que le solvant est éliminé par distillation.

20. Procédé selon l'une des revendications précédentes caractérisé en ce que le produit réactionnel de l'étape de procédé (b) est repris en phase aqueuse et acidifié à un pH entre 2,0 et 9,0.

21. Procédé selon la revendication 20 caractérisé en ce que l'on acidifie à un pH de 3,5 à 5,5.

22. Procédé selon la revendication 21 caractérisé en ce qu'un acide minéral ou carboxylique est utilisé pour l'acidification.

23. Procédé selon la revendication 22 caractérisé en ce que l'acide chlorhydrique, l'acide sulfurique ou l'acide acétique est utilisé.

24. Procédé selon l'une des revendications 20 à 23 caractérisé en ce que, dans une étape de traitement consécutive, on lave le produit sous forme de solide avec de l'eau pour le débarrasser des sels et, éventuellement, on le sèche.
